# EUROPEAN PATENT SPECIFICATION

(11) **EP 3 318 281 B1**
(45) Date of publication and mention of the grant of the patent: **21.12.2022**
(21) Application number: 16197420.9
(22) Date of filing: 04.11.2016
(51) Int. Cl.: A61K 47/26, A61K 35/00, A61K 39/395, A61K 9/19

(54) **HIGHLY PURIFIED SUGARS AND SUGAR COMPOSITIONS**
HOCHAUFGEREINIGTE ZUCKER UND ZUCKERZUSAMMENSETZUNGEN
SUCRES ET COMPOSITIONS DE SUCRE HAUTEMENT PURIFIÉES

(43) Date of publication of application: 09.05.2018
(73) Proprietor: Coriolis Pharma Research GmbH, 82152 Martinsried (DE)
(72) Inventor: WEINBUCH, Daniel, 82152 Martinsried (DE); HAWE, Andrea, 82152 Martinsried (DE); JISKOOT, Wim, deceased (NL)
(74) Representative: Pharma Patents International AG

(56) References cited:
- WO-A1-01/36690
- WO-A1-2015/191110
- US-A1- 2004 241 174
- US-A1- 2014 072 559
- WEINBUCH DANIEL ET AL: "Nanoparticulate Impurities in Pharmaceutical-Grade Sugars and their Interference with Light Scattering-Based Analysis of Protein Formulations", PHARMACEUTICAL RESEARCH, SPRINGER NEW YORK LLC, US, vol. 32, no. 7, 30 January 2015 (2015-01-30), pages 2419-2427, XP035927593, ISSN: 0724-8741, DOI: 10.1007/S11095-015-1634-1 [retrieved on 2015-01-30]

## Description

### FIELD

The invention relates to sugars, in particular sucrose, their use in pharmaceutical compositions, and further to nanoparticulate impurities comprised in sugars. The invention further relates to methods for obtaining sucrose with reduced levels of the nanoparticulate impurities and related methods of preparing pharmaceutical compositions.

### BACKGROUND

As described in Weinbuch et al.; "Nanoparticulate Impurities in Pharmaceutical-Grade Sugars and their Interference with Light Scattering-Based Analysis of Protein Formulations"; Pharm Res. 2015 Jul; 32(7):2419-27, it was recently found that pharmaceutical-grade sugars such as sucrose contain nanoparticulate impurities (NPI), i.e. nanoparticles in the size range of typically about 100-200 nm in average, which unlike sucrose do not dissolve in the aqueous medium. These impurities give rise to an interference signal at 100-200 nm that is commonly observed with aqueous sugar-containing solutions during e.g. dynamic light scattering (DLS) and nanoparticle tracking analysis (NTA) measurements. The signal is not caused by the dissolved sugar molecules whose size is only about 0.5 nm and 1 nm for mono- and disaccharides, respectively. Other authors had also noticed this interference signal in the past, but did not investigate or identify its cause or potential effects on pharmaceutical compositions.

The intensity of the interference signal and thus the amount, or count of NPI particles, differs between sugar types (e.g. trehalose, fructose maltose and galactose), sucrose of various purity grades, obtained from different suppliers, and different batches of the same supplier; the particle size distribution on the other hand does not vary much.

Weinbuch et al. further showed that the nanoparticles present in sucrose are apparently agglomerates composed of various impurities originating from raw materials, such as dextrans, ash components and aromatic colorants. It is unclear whether these NPI form spontaneously upon dissolution in an aqueous medium (as usually present during DLS and NTA analysis), or whether they are already present in their nanoparticulate form in the dry solid sucrose. Furthermore, Weinbuch et al. is silent on any endotoxin-like properties of the nanoparticles found.

The same considerations apply to, for instance, WO01/36690A1. While being aimed at removing invert sugars from a sucrose-containing feed solution by nanofiltration, WO01/36690A1 is altogether silent on water-insoluble nanoparticulate impurities exhibiting a particle size of 20-500 nm and/or endotoxin-like properties.

In fact, the presence of water-insoluble NPI with endotoxin-like properties, and exhibiting a particle size in the range of 20-500 nm, in sugars, even pharmaceutical-grade sugars, was completely unknown to the skilled person prior to the present invention.

And while WO2015/191110A1 addresses the removal of endotoxins in the narrower sense (i.e., lipopolysaccharides) from aqueous sugar solutions using anion exchange chromatography in a polyethyleneimine (PEI) column, said endotoxins are neither explicitly nor implicitly described to be water-insoluble nanoparticles of any specific size.

As apparent from Weinbuch et al., WO01/36690A1 and WO2015/191110A1, sugars may comprise a variety of impurities without any structural and/or compositional relationship to one another. This is problematic considering the use of sugars in pharmaceutical compositions.

Sugars, and in particular sucrose and trehalose, are often employed in pharmaceutical compositions comprising polypeptide-based biologically active ingredients because they are preferentially excluded from the protein's surface, thereby increasing the free energy of the system and promoting conformational stability. Furthermore, sugars such as sucrose are used extensively as cryoprotectors and lyoprotectors for lyophilized protein compositions.

The overall impact of the NPI on the biological activity, performance or stability of pharmaceutical compositions with polypeptide-based active ingredients has not been investigated in the past, and sugars including sucrose that comply with the specifications defined in the respective pharmacopoeial monographs have been widely used and incorporated in such compositions irrespective of their NPI content.

The present invention is based on the inventors' discovery that NPI may present a significant threat to the performance and stability of pharmaceutical compositions comprising sucrose and without control of the NPI content there is a substantial risk of not being able to manufacture batches of such compositions which have consistent quality.

### SUMMARY OF THE INVENTION

In a first aspect, the invention provides a method of preparing ultrapure sucrose, the method comprising the steps of (b) providing a quantity of a first sucrose comprising water-insoluble nanoparticulate impurities (NPI) at a level of 10⁶ nanoparticles per gram of sucrose or higher, wherein the nanoparticles have a particle size ranging from 20 nm to 500 nm, comprise a (1-3)-β-glucan, and exhibit endotoxin-like properties; wherein prior to step (b), a step (a) is conducted of obtaining or determining the level of NPI in the first sucrose; and then (c) reducing the level of NPI to 10⁵ nanoparticles per gram of sucrose or lower by (i) separating at least some of the NPI from the sucrose, optionally by filtration, centrifugation, crystallisation or chromatography of a solution of said sucrose; or by (ii) diluting the first sucrose with a second sucrose, the second sucrose comprising NPI at a level below 10⁵ nanoparticles per gram of sucrose. Optionally, the method may further comprise a step (d) of concentrating the sucrose solution of step (i) to a concentration of 10 % (w/v) or higher, or of drying the sucrose solution such as to obtain dry solid sucrose. Moreover, the method may further comprise a step of obtaining or determining the level of NPI in the first sucrose, optionally by nanoparticle tracking analysis (NTA).

As stated above, the water-insoluble NPI particles have a particle size ranging from 20 nm to 500 nm. Furthermore, the NPI may have a particle size distribution (PSD) characterised by a D50 value from 80 nm to 300 nm, in particular from 100 nm to 200 nm, as measured by nanoparticle tracking analysis (NTA) of an aqueous solution of the sucrose.

As further stated above, the water-insoluble NPI particles further exhibit endotoxin-like properties; i.e. they provide a positive test result in endotoxin tests such the Limulus Amebocyte Lysate tests (LAL) and/or modified LAL tests. More specifically, the NPI comprise an immune-modulating (1-3)-β-glucan, as may be determined, for instance, by a modified LAL tests using reagents with eliminated Factor C, such as the (1-3)-β-glucan specific Glucatell^{®} test kit.

The NPI may further comprise a dextran, optionally crosslinked and/or exhibiting a mean molecular mass from 10 kDa to 100 kDa. Moreover, the NPI may comprise one or more organic colourants and/or an inorganic compound selected from silicium, aluminium, calcium, magnesium, phosphorus, sulphur, potassium, iron, or any combination of any of these.

The method may be conducted such as to reduce the level of NPI to not more than 10³ nanoparticles per gram of sucrose, or not more than 10² nanoparticles per gram of sucrose; or even such that the sucrose is substantially free of NPI, as can be determined by any suitable light-scattering based analytical techniques, such as nanoparticle tracking analysis (NTA) or electric zone sensing using a so-called Coulter counter.

Where the level of NPI is reduced via filtration or centrifugation, respectively, this may be performed by subjecting an aqueous solution of the sucrose to an ultrafiltration or ultradiafiltration process or to ultracentrifugation, respectively. Optionally, ultrafiltration may be performed through a filter with a pore size of 0.02 µm or smaller.

In a second aspect, the invention relates to an ultrapure sucrose as obtainable by the method described above. The invention further provides a solid, crystalline sucrose comprising water-insoluble nanoparticulate impurities (NPI) at a level of not more than 10⁵ nanoparticles per gram of sucrose as determined by nanoparticle tracking analysis (NTA), preferably not more than 10³ nanoparticles per gram of sucrose, and more preferably not more than 10² nanoparticles per gram of sucrose.

In a third aspect, the invention relates to the use of such ultrapure sucrose for the preparation of a pharmaceutical or diagnostic composition comprising an active ingredient, preferably an active ingredient such as a peptide, a protein, a conjugate comprising a peptide or protein, a lipopeptide, or a lipoprotein; an active ingredient prone to aggregation and/or degradation; and/or an active ingredient sensitive to heat, mechanical stress, oxygen, acidic and/or basic conditions.

In a further aspect, the invention relates to a method of preparing a pharmaceutical or diagnostic composition comprising an active ingredient and sucrose, the method comprising the steps of obtaining or determining the level of NPI in the sucrose, optionally by nanoparticle tracking analysis (NTA); then, if the level of NPI in the sucrose is 10⁶ nanoparticles per gram of sucrose or higher, or if the level of NPI in the sucrose is such that it would result in a level of NPI of 10⁵ nanoparticles per gram of composition or higher, reducing the level of NPI to 10⁵ nanoparticles per gram of sucrose or lower by (i) separating at least some of the NPI from the sucrose, optionally by filtration, centrifugation, crystallisation or chromatography; or by (ii) diluting the first sucrose with a second sucrose, the second sucrose comprising NPI at a level below 10⁵ nanoparticles per gram of sucrose; and finally preparing the pharmaceutical or diagnostic composition incorporating the sucrose.

This method is particularly useful when preparing a plurality of consecutive batches of the pharmaceutical or diagnostic composition in the described manner, optionally using different batches of sucrose. Advantageously, the method may result in a higher reproducibility in terms of quality, purity and stability of the batches than currently achievable with sucrose-containing compositions.

The pharmaceutical or diagnostic composition prepared with this method may, for instance, be in the form of a powder or a lyophilised solid foam for reconstitution, or in the form of an aqueous liquid.

In a yet further aspect, the invention relates to a pharmaceutical or diagnostic composition as obtained by the above described preparation method for use in the diagnosis or therapy of a human or a non-human animal. For instance, the pharmaceutical or diagnostic composition may comprise a peptide, a protein, a conjugate comprising a peptide or protein, a lipopeptide, or a lipoprotein as the active ingredient; and/or an active ingredient prone to aggregation and/or degradation; and/or an active ingredient sensitive to heat, mechanical stress, oxygen, acidic and/or basic conditions. In one embodiment, the pharmaceutical or diagnostic composition may comprise a monoclonal antibody, and/or the sucrose in the pharmaceutical or diagnostic composition is substantially free of NPI, as determined by nanoparticle tracking analysis (NTA).

### DEFINITIONS

All technical terms as used herein shall be understood to have the same meaning as is commonly understood by a person skilled in the relevant technical field. Furthermore, the following terms or expressions as used herein should normally be interpreted as outlined in this section, unless defined otherwise by the description or unless the specific context indicates or requires otherwise:
The words 'comprise', 'comprises' and 'comprising' and similar expressions are to be construed in an open and inclusive sense, as 'including, but not limited to' in this description and in the claims.

The singular forms 'a', 'an' and 'the' should be understood as to include plural referents. In other words, all references to singular characteristics or limitations of the present disclosure shall include the corresponding plural characteristic or limitation, and vice versa. The terms 'a', 'an' and 'the' hence have the same meaning as 'at least one' or as 'one or more'. For example, reference to 'a nanoparticle' includes a plurality of nanoparticles, and the like.

The expressions, 'one embodiment', 'an embodiment', 'a specific embodiment' and the like mean that a particular feature, property or characteristic, or a particular group or combination of features, properties or characteristics, as referred to in combination with the respective expression, is present in at least one of the embodiments of the invention. These expressions, occurring in various places throughout this description, do not necessarily refer to the same embodiment. Moreover, the particular features, properties or characteristics may be combined in any suitable manner in one or more embodiments.

All percentages, parts and/or ratios in the context of numbers should be understood as relative to the total number of the respective items, unless otherwise specified, or indicated or required by the context. Furthermore, all percentages parts and/or ratios are intended to be by weight of the total weight; e.g. '%' should be read as 'wt.-%', unless otherwise specified (e.g. w/v), or indicated or required by the context.

'Essentially', 'about', 'approximately' (approx.), 'circa' (ca.) and the like in connection with an attribute or value include the exact attribute or the precise value, as well as any attribute or value typically considered to fall within a normal range or variability accepted in the technical field concerned.

Any reference signs in the claims should not be construed as a limitation to the embodiments represented in any of the drawings.

As used herein, nanoparticulate impurities (NPI) means impurities which are in an aqueous environment, in particular at a physiologically compatible pH, such as within the range of pH 4 to pH 9 or from pH 5 to pH 8, detectable as nanoparticles having a particle size of 20 nm to 500 nm, e.g., as determined by laser diffraction or dynamic light scattering. Moreover, the NPI nanoparticles are characterised by exhibiting an endotoxin-like immunoreactivity, which may be detected or determined, for example, with a Limulus test, also known as Limulus Amebocyte Lysate (LAL) test, or by any other test for identifying endotoxin-like properties, such as a modified LAL-test in which the Factor C is eliminated (e.g. Glucatell^{®} test). While endotoxins are, according to a narrow definition, lipopolysaccharides found in the outer membrane of Gram-negative bacteria which trigger immune responses in animals, also other compounds which may not necessarily be derived from bacteria only (such as (1-3)-β-glucan which is also found in yeast or fungi) may exhibit immune-modulating properties and can be detected and quantified e.g. with the Glucatell^{®} test. Both bacterial endotoxins and (1-3)-β-glucan are also referred to as 'Pathogen-Associated Molecular Patterns' (PAMPs) in the literature.

With respect to the nanoparticles, water-insoluble means that the substance or substances forming the NPI nanoparticles are substantially less water-soluble than the sucrose, for which reason they are detectable as nanoparticles in an aqueous environment.

As used herein, ultrapure with respect to sucrose means that the sucrose exhibits a high degree of purity, also with respect to the level of NPI. The expression may also be understood as meaning that the sucrose does not incorporate NPI at a level which would detrimentally affect the quality, performance or stability of a pharmaceutical formulation comprising the sucrose. Sucrose comprising NPI at a level of not more than about 10⁶ nanoparticles per g may also be considered as an ultrapure sucrose.

Nanoparticulate, as used herein, refers to any type, form, structure or morphology of nanoparticle that may exist, in particular in an aqueous environment.

### DETAILED DESCRIPTION OF THE INVENTION

In a first aspect, the present invention provides a method of preparing ultrapure sucrose, the method comprising the steps of:
(b) providing a quantity of a first sucrose comprising water-insoluble nanoparticulate impurities (NPI) at a level of 10⁶ nanoparticles per gram of sucrose or higher, wherein the nanoparticles have a particle size ranging from 20 nm to 500 nm, comprise a (1-3)-β-glucan, and exhibit endotoxin-like properties;
wherein prior to step (b), a step (a) is conducted of obtaining or determining the level of NPI in the first sucrose;
(c) reducing the level of NPI to 10⁵ nanoparticles per gram of sucrose or lower by
   (i) separating at least some of the NPI from the sucrose, optionally by filtration, centrifugation, crystallisation or chromatography of a solution of said sucrose; or
   (ii) diluting the first sucrose with a second sucrose, the second sucrose comprising NPI at a level below 10⁵ nanoparticles per gram of sucrose, and optionally (d) concentrating the sucrose solution of step (i) to a concentration of 10 % (w/v) or higher, or drying the sucrose solution such as to obtain dry solid sucrose.

The inventors have discovered that the NPI present in sucrose, even at a level at which the sucrose complies with all specifications provided by the major pharmacopoeias, may negatively affect the performance and stability of pharmaceutical compositions with polypeptide-based active ingredients. Moreover, the inventors found that in particular the batch-to-batch consistency of the performance and stability of such compositions may be adversely affected by variable NPI levels of sucrose. In the absence of such insights, it was entirely unexpected that by controlling the NPI content of the sucrose, e.g. by selecting a sucrose that has a low NPI content or by purifying sucrose with respect to the NPI content prior to its incorporation within a pharmaceutical composition, the product quality of the pharmaceutical compositions and its batch-to-batch consistency could be significantly increased.

Optionally, the step (a) of obtaining or determining the level of NPI in the first sucrose, which is conducted prior to step (b)is conducted by nanoparticle tracking analysis (NTA), as detailed for instance in the examples section below; however, any other particle size determination method which is capable of sensing and quantifying particles in the size range of 20 nm up 500 nm may be used as well, e.g. other light-scattering based analytical techniques like dynamic light scattering (DLS).

In one embodiment, the NPI particles have a particle size distribution in an aqueous solution characterised by a D50 value from 80 nm to 300 nm as measured by nanoparticle tracking analysis (NTA), in particular from 100 nm to 200 nm, or from 125 nm to 165 nm.

In a further embodiment, the D90 value is from about 100 nm to about 400 nm as measured by nanoparticle tracking analysis (NTA), in particular from about 150 nm to about 350 nm, or from about 220 nm to about 290 nm. Alternatively, the D10 value is from about 50 nm to about 150 nm as measured by nanoparticle tracking analysis (NTA), in particular from about 70 nm to about 130 nm, or from about 80 nm to about 110 nm.

In a further embodiment, the particle size distribution of the NPI particles is monomodal.

As stated above, the NPI comprise a (1-3)-β-glucan. This type of compound - which may be derived from the cell walls of bacteria, yeast and fungi - may contribute, or even be primarily responsible for the immunomodulatory or endotoxin-like properties of the NPI. This is one of the reasons why the level of NPI in sucrose, but also in pharmaceutical compositions comprising the sucrose, should be kept as low as possible. The presence and content of (1-3)-β-glucan in the NPI can be determined, for example, by a modified Limulus Amebocyte Lysate (LAL) test using a reagent with the Factor C being eliminated which renders this test more specific for (1-3)-β-glucan. This test is detailed, for instance, in the examples section below. Without wishing to be bound by theory, it is possible that the (1-3)-β-glucans found in the NPI stems at least partially from the cell walls of microorganisms, e.g. Leuconostoc bacteria, which enter the sugar cane or beet e.g. during harvesting, cutting and grinding but also during later processing steps; however they may also stem from other sources such as fungi or yeast.

Based on the discovery by the inventors that a (1-3)-β-glucan may be present in sucrose-derived NPI nanoparticles, the method of the invention is particularly advantageous and leads to unexpected improvements in product quality, performance and stability for any pharmaceutical composition for whose manufacture the incorporation of a sucrose comprising such NPI is contemplated.

In one embodiment, the NPI comprise a dextran, wherein the dextran is optionally crosslinked and/or exhibits a mean molecular mass from 10 kDa to 100 kDa. In a specific embodiment, the NPI comprise a (1-3)-β-glucan and a dextran, wherein the dextran is optionally crosslinked and/or exhibits a mean molecular mass from 10 kDa to 100 kDa

Moreover, the NPI may comprise one or more organic colourants and/or an inorganic compound selected from silicium, aluminium, calcium, magnesium, phosphorus, sulphur, potassium, iron, or any combination of any of these. In a specific embodiment, the colourants are aromatic colourants, as may be detected or determined, for instance, by measuring the internal fluorescence of a sample. In a more specific embodiment, the colourants are aromatic colourants exhibit two patterns of maximum fluorescence intensity; pattern 1 at about 280/390 nm (which may potentially be caused by two fluorophores with a close similarity to tryptophan and tyrosine) and pattern 2 at about 340/420 nm (which may potentially be caused by catechols formed by base-catalysed sugar degradation).

In one embodiment of the method to prepare ultrapure sucrose, step (c) is conducted such as to reduce the level of NPI to not more than 10³ nanoparticles per gram of sucrose, or not more than 10² nanoparticles per gram of sucrose; or such that the sucrose is substantially free of NPI, as determined by light-scattering based analytical techniques, such as dynamic light scattering (DLS) or nanoparticle tracking analysis (NTA). In that regard, it should be understood that 'substantially free of NPI' means, that no NPI can be detected according to currently available methods; however, this does not exclude that a small number of particles below the level of detection of the respective method, e.g. NTA, may nonetheless be present.

In one embodiment, step (c) may be performed such as to reduce the level of NPI particles per g of sucrose by the factor of 100 or higher. Optionally, step (c) is performed such as to reduce the NPI particles by the factor of at least 200, or at least 300, or at least 500, or at least 1,000, or at least 2,000, or at least 3,000, or at least 5,000, or at least 10,000, respectively.

In one embodiment, the filtration in step (c)(i) is performed as an ultrafiltration or ultradiafiltration of an aqueous solution of the first sucrose. Optionally, the aqueous solution of the first sucrose may be subjected to ultrafiltration through a filter with a pore size of approx. 0.02 µm or smaller, such as a PVDF filter with a nominal pore size down to 0.001 µm; or through a membrane with a cut-off of 30 kDa or smaller. Filtration through a 0.02 µm filter decreases the NPI related signal in both DLS and NTA measurements to background levels, i.e. not showing a peak at about 100-200 nm anymore. Filters with larger pore sizes, such as common sterile filtration filters (often 0.2 µm or 0.22 µm) or even 0.1 µm filters were found to be substantially less efficient in reducing the level of NPI in aqueous sucrose solutions.

In a specific embodiment or as an alternative to ultrafiltration by syringe filters, the aqueous solution of the first sucrose may be subjected to ultradiafiltration in a tangential flow filtration (TFF) system equipped with a TFF-membrane; for instance, a TFF-membrane with a cut-off of 30 kDa or smaller. Such ultradiafiltration systems have been used by the inventors in order to obtain and/or purify NPI from aqueous sucrose solutions, e.g. in order to investigate the size and composition of NPI and /or their level per gram sucrose.

In a further embodiment, the centrifugation in step (c)(i) is performed as ultracentrifugation of an aqueous solution of the first sucrose. Optionally, centrifugal filter units may be employed during ultracentrifugation, e.g. units equipped with a filter membrane having a cut-off of 30 kDa or 10 kDa. Such centrifugal filter units have also been used by the inventors in order to obtain and/or purify NPI from aqueous sucrose solutions, in particular for concentrating NPI-containing samples after ultradiafiltration.

As described above for step (c)(ii), a sucrose of acceptable NPI level (i.e. max. 10⁵ nanoparticles per gram of sucrose) may also be obtained by diluting the first sucrose with a second sucrose, the second sucrose comprising NPI at a level below 10⁵ nanoparticles per gram of sucrose; or in other words by mixing a 'high-NPI sucrose' with a 'low-NPI sucrose' until the total content of the mixture falls below 10⁵ nanoparticles per gram of sucrose.

It will be understood that typically this dilution step is advisable and economically reasonable only in cases where the second sucrose exhibits an NPI-content sufficiently low to 'counter-act' the higher NPI-content in the first sucrose. For instance, if the first and second sucrose comprise 10⁶ and 10¹ nanoparticles per gram of sucrose, respectively, a 1:10 mixture would suffice to reduce the NPI in the mixture to an acceptable level of below 10⁵ nanoparticles per gram of sucrose. This reasonable dilution or mixing ratio would allow manufacturers of pharmaceutical grade sugars - within given boundaries - to account for inherent differences in their raw materials which may have an effect on the final NPI level. For instance, the dextran content in the raw material is typically guided by the load of Leuconostoc bacteria as described earlier.

However, if the first sucrose comprises a higher number of NPI, such as 10⁹ nanoparticles per gram of sucrose already (a content as found in pharmaceutical grade sucrose by the inventors in an earlier study), even blends with a substantially NPI-free second sucrose would require very large amounts of said second sucrose; in which case it may be economically more reasonable to remove or reduce these NPI from the first sucrose by one of the other steps, such as filtration, centrifugation, crystallisation or chromatography, rather than attempting a dilution.

In a further aspect, the invention also provides the sucrose as obtainable by the method(s) described above; i.e. a purified sucrose from which the NPI were substantially removed or in which the level of NPI has been reduced to 10⁵ nanoparticles per gram of sucrose or lower. Such ultrapure sucrose offers a number of advantages, in particular when incorporated into pharmaceutical or diagnostic compositions. For instance, ultrapure sucrose introduces less NPI and in consequence less (1-3)-β-glucan (as comprised in the NPI) and less nanoparticles which could potentially act as an initial nucleus, or nucleation site, for crystallisation and/or aggregation processes, especially in aqueous compositions. Thereby stability and shelf-life of (aqueous) compositions is improved and the risk of immunogenic reactions decreased. Also, since the inventors report that the level of NPI found in sucrose varies distinctly, removing or at least reducing the level of NPI to the level of 10⁵ nanoparticles per gram of sucrose or lower, helps to ensure and/or improve the batch-to-batch reproducibility during preparation of pharmaceutical or diagnostic compositions; i.e. a plurality of consecutive batches prepared with sucrose will exhibit less variability in terms of quality, purity and stability parameters, such as content and activity of the drug or number of aggregates formed at time to (after preparation) and after times t₁ to tₙ during storage under ambient and/or accelerated conditions.

The same advantages will apply to ultrapure sucrose comprising not more than 10⁵ nanoparticles per gram of sucrose *per se,* i.e. to a sucrose which contains limited levels of NPI as an outcome of e.g. an optimized sugar refinement process and/or low initial levels of NPI in the raw materials from which the sucrose is derived. Hence, in a similar aspect, the invention also provides ultrapure, solid, crystalline sucrose comprising water-insoluble nanoparticulate impurities (NPI) at a level of not more than 10⁵ nanoparticles per gram of sucrose as determined by nanoparticle tracking analysis (NTA). In preferred embodiments, the ultrapure, solid, crystalline sucrose comprises not more than about 10⁴ nanoparticles per gram of sucrose, not more than about 10³ nanoparticles per gram of sucrose; or not more than about 10² nanoparticles per gram of sucrose, respectively.

In a yet further aspect, the invention provides the use of ultrapure sucrose (i.e. sucrose comprising not more than 10⁵ nanoparticles per gram of sucrose) for the preparation of a pharmaceutical or diagnostic composition comprising an active ingredient, or drug. As described above, the use of ultrapure sucrose offers number of benefits when incorporated in such compositions. Again, it is also preferred that the ultrapure sucrose comprises not more than about 10⁴ nanoparticles per gram of sucrose, not more than about 10³ nanoparticles per gram of sucrose; or not more than about 10² nanoparticles per gram of sucrose, respectively.

Preferably, the active ingredient in these pharmaceutical or diagnostic compositions is (a) a peptide, a protein, a conjugate comprising a peptide or protein, a lipopeptide, or a lipoprotein; (b) prone to aggregation and/or degradation; and/or (c) sensitive to heat, mechanical stress, oxygen, acidic and/or basic conditions. Proteins have an inherent propensity to aggregate (and ultimately precipitate) because their structure is dynamic and mainly held together by a combination of van der Waals forces, disulfide linkages, hydrogen bonds, etc., and as aggregation proceeds, the activity of the protein molecules may diminish or be lost, thereby reducing the efficacy of the composition. Furthermore, it is known from studies that the presence of protein aggregates in a composition (especially when intended for parenteral administration) may trigger unwanted and/or potentially dangerous immune responses in the recipient. Among other factors, the presence of contaminating particles such as silicone oil droplets or silicone rubber from container-closure systems is already known to promote protein aggregation; and apparently the very same applies to the NPI as the inventors have found out when using e.g. antibodies as model drugs (see e.g. the examples further below). Therefore, compositions comprising proteins and/or other active ingredients prone to aggregation and/or degradation may benefit in particular from the use of ultrapure sucrose.

With respect to the active ingredient it should be understood that in some cases also the sucrose itself may be considered as the active ingredient, or at last as one of the active ingredients; for instance, where the pharmaceutical composition is an oral solution (such as a 24 % sucrose oral solution for pain relief in neonates) or a composition for parenteral nutrition. In particular the latter may benefit from the use of ultrapure sucrose, since the (1-3)-β-glucan related immunogenicity is typically more pronounced with parenteral than with oral compositions.

In a further aspect, the invention therefore provides a method of preparing a pharmaceutical or diagnostic composition comprising an active ingredient and sucrose, the method comprising the steps of
(a) obtaining or determining the level of NPI in the sucrose, optionally by nanoparticle tracking analysis (NTA);
(b) if the level of NPI in the sucrose is 10⁶ nanoparticles per gram of sucrose or higher, or if the level of NPI in the sucrose is such that it would result in a level of NPI of 10⁵ nanoparticles per gram of composition or higher, reducing the level of NPI to 10⁵ nanoparticles per gram of sucrose or lower by
   (i) separating at least some of the NPI from the sucrose, optionally by filtration, centrifugation, crystallisation or chromatography; or
   (ii) diluting the first sucrose with a second sucrose, the second sucrose comprising NPI at a level below 10⁵ nanoparticles per gram of sucrose; and
(c) preparing the pharmaceutical or diagnostic composition incorporating the sucrose.

In one embodiment, the pharmaceutical or diagnostic composition prepared by the above method is provided in the form of (a) a powder or a lyophilised solid foam for reconstitution, or (b) an aqueous liquid. With regard to the nanoparticle content per gram of composition, it should be understood, that the provided level of NPI refers to the final composition; i.e. in the case of a powder or a lyophilised solid foam for reconstitution it is intended to refer to the reconstituted composition. The sucrose content in common, currently marketed products for reconstitution such as Enbrel^{®}, Serostim^{®}, Remicade^{®} or Stelara^{®} is typically below 10 % in the reconstituted solutions, ranging from about 1 % to about 8 %, such that it is advisable to reduce the level of NPI in the composition below 10⁵ nanoparticles per gram of composition, preferably below 10⁴ nanoparticles per gram of composition and more preferably below 10³ nanoparticles per gram of composition.

In a specific embodiment, the level of NPI will be reduced to 10³ nanoparticles per gram of sucrose or lower using the above mentioned steps (i) or (ii) if the level of NPI in the sucrose is such that it would result in a level of NPI of 10⁵ nanoparticles per gram of composition or higher.

In a specific embodiment, the separation of the NPI from the sucrose in step (i) of the above preparation method by filtration, centrifugation, crystallisation or chromatography is performed such as to reduce the number of nanoparticles below the detection limit of the respective method, e.g. NTA; i.e. such that the sucrose is substantially free of NPI when incorporating it into the pharmaceutical or diagnostic composition along with the active ingredient.

With respect to the NPI levels chosen herein in terms of maximum levels accepted per unit of sucrose or per unit of a composition (or desirable levels to meet or preferably fall below), it should be understood that these levels are predominantly provided as guidance and may be adapted to specific situations or products by the skilled person. It will be easily understood that a composition which contains only rather low amounts of sucrose, such as about 2 % or lower, may tolerate a higher NPI level than a composition with rather high sucrose contents, such as about 10 % or higher. Similarly, a composition comprising aggregation sensitive compounds such as proteins or comprising supersaturated drugs prone to precipitation may be more sensitive to the NPI level of the sucrose used than other compositions in which the NPI are less likely to trigger aggregation or crystallisation. Also, a paediatric composition intended for neonates or infants may require smaller NPI levels than compositions for adults, since infants will typically react more sensitive to endotoxins such as the (1-3)-β-glucan comprised in the NPI.

In one embodiment, a plurality of consecutive batches of the pharmaceutical or diagnostic composition is prepared, and each of these batches is prepared following the steps of the above described method. Optionally, different batches of sucrose may be used.

Preparing a plurality of consecutive batches (rather than e.g. just one patient specific batch) is very common in the field of preparing pharmaceutic or diagnostic compositions. It is also common that different batches, or lots, of the required raw materials are used. As the inventors have shown, the level of NPI found in sucrose varies. Hence, removing or reducing the level of NPI to 10⁵ nanoparticles per gram of sucrose or even lower helps to ensure and/or improve the batch-to-batch reproducibility of the pharmaceutical or diagnostic compositions prepared. In consequence, the plurality of consecutive batches will exhibit less variability in terms of quality, purity and stability parameters, such as content and activity of the drug or number of aggregates formed at time to (after preparation) and after times t₁ to tₙ during storage under ambient and/or accelerated conditions.

In a further aspect, the invention therefore provides a pharmaceutical or diagnostic composition as obtained by the above preparation method for use in the diagnosis or therapy of a human or a non-human animal.

In one embodiment, the active ingredient used in the diagnosis or therapy is (a) a peptide, a protein, a conjugate comprising a peptide or protein, a lipopeptide, or a lipoprotein; (b) an active ingredient prone to aggregation and/or degradation; and/or (c) an active ingredient sensitive to heat, mechanical stress, oxygen, acidic and/or basic conditions.

In a specific embodiment, the active ingredient in the pharmaceutical or diagnostic composition is a monoclonal antibody; and/or the sucrose pharmaceutical or diagnostic composition is substantially free of NPI, as determined by nanoparticle tracking analysis (NTA).

### EXAMPLES

### Example 1: Sucrose derived NPI and their effect on stability of monoclonal antibodies

Herceptin^{®} (trastuzumab), MabThera^{®} (rituximab), Remicade^{®} (infliximab), and Erbitux^{®} (cetuximab) were donated by local hospitals. Pharmaceutical-grade sucrose (Ph.Eur.) was purchased from VWR BDH Prolabo^{®} (Bruchsal, Germany) and Südzucker (Mannheim, Germany). Hisitidine-HCl, trehalose, sodium citrate, sodium chloride, sodium dihydrogen phosphate, di-sodium hydrogen phosphate, and citric acid were purchased from Merck (Darmstadt, Germany). Polysorbate 20 and 80, and glycine was purchased from Sigma (Taufkirchen, Germany). Histidine was purchased from Amresco (Solon, Ohio, USA). PVDF syringe and membrane filters with a pore size of 0.2 µm were obtained from Millipore (Schwalbach, Germany).

NPI particles were obtained from pharmaceutical-grade sucrose batches by diafiltration of 1 L freshly prepared aqueous sucrose solution 50 % (w/v) using a Minimate II Tangential Flow Filtration (TFF) system equipped with a 30 kDa TFF membrane (both from Pall, Crailsheim, Germany). The retentate was concentrated to about 300 mL and subsequently diafiltrated with MilliQ^{®}-water until a diafiltration volume DV of 14 was achieved (ratio between filtrate volume and retentate volume). Next, the retentate was concentrated to about 50 mL and filtered with a syringe filter (0.2 µm) before the retentate was further concentrated to about 1 mL with Amicon Ultra 15 centrifugal filter-units (Millipore) with a molecular cut-off of 10 kDa. The NPI sample was aliquoted into 0.5 mL cryotubes and finally stored at -80 °C until further use. The final sample contained 7×10¹¹ nanoparticles/mL, the particles exhibiting a size range of about 100-200 nm and a monomodal PSD, as determined by NTA (see below).

For the nanoparticle tracking analysis (NTA) of the NPI particles, a NanoSight LM20 (NanoSight, Amesbury, UK) instrument equipped with a 405 nm blue laser, a sample chamber and a Viton fluoroelastomer O-ring was employed. Using a 1 mL syringe and a pre-run volume of 0.5 mL, samples are loaded into the sample chamber and subsequently analysed in triplicate at stopped flow. Between each repetition, 0.1 mL of sample is flushed through the chamber. The NTA 2.3 software is used for capturing and analysing the data; and movements of the particles in the samples are recorded as videos for 60 s, while the shutter and gain settings of the camera are set automatically by the software for an optimal particle resolution. If sample dilution is necessary to achieve a more optimised concentration for NTA, ultrapure water of Type 1 as defined in e.g. ISO 3696 (such as Milli-Q^{®} water or MQ-water) is used as a diluent and all results calculated back to the original concentration.

The zeta-potential of the NPI particles was measured by laser Doppler electrophoresis (LDE) by using a Zetasizer Nano ZS (Malvern Instruments, Worcestershire, UK) and the Zetasizer software version 7.03 for system control and data acquisition. NPI samples at a concentration of 5 × 10¹¹ nanoparticles /mL (based on NTA) were buffered at pH 7.4 (1 mM phosphate buffer) or at pH 3.0 (1 mM citrate buffer) and 1000 µL sample transferred into a folded capillary cell (Malvern) for measurement. Each zeta-potential measurement is the average of three measurements consisting of 100 sub runs. The LDE evaluation reconfirmed earlier results in that the NPI particles exhibited a zeta potential of -13.4 ± 1.6 (at pH 7.4) and -8.0 ± 0.3 mV (at pH 3.0).

The (1-3)-β-glucan level in the NPI particles is measured using a Glucatell^{®} kit (Cape Cod, East Falmouth, USA), according to the instructions of the manufacturer. For this purpose, the NPI containing samples are diluted with endotoxin free water in four 10-fold serial dilution steps under aseptic and particle free conditions. The mixture of the (diluted) sample and the Glucatell^{®}-reagent is placed in a microplate-heating block at 37 °C for the recommended time. A volume of 50 µL from each of the three diazo reagents is added to the mixture to stop the reaction. The absorbance at 545 nm is measured and (1-3)-β-glucan concentration in pg/mL is calculated based on a standard curve. The NPI particles gave a strong signal for (1-3)-β-glucan, namely 750 ng/mL for the NPI sample with about 7 × 10¹¹ nanoparticles/mL, compared to only 0.013 ng/mL for the control sample.

Trastuzumab, rituximab, infliximab, and cetuximab were diluted to a monoclonal antibody (mAbs) concentration of 2 mg/mL using the following formulation buffers (all filtered through 0.2 µm PVDF syringe filters):
Trastuzumab: 2.4 mM histidine-HCl, 2.1 mM histidine, 52.9 mM trehalose, and 0.009 % polysorbate 20 at pH 6.0.
Rituximab: 25 mM citrate buffer, 9 g/L sodium chloride, and 0.7 g/L polysorbate 80 at pH 6.5.
Infliximab: 5 mM phosphate buffer, 50 g/L sucrose (Südzucker), and 0.005 % polysorbate 80 at pH 7.2.
Cetuximab: 10 mM citric acid buffer, 100 mM sodium chloride, 100 mM glycine, and 0.01 % polysorbate 80 at pH 5.5.

Trastuzumab, rituximab, infliximab, and cetuximab, as well as their corresponding formulation buffers, were spiked with NPI to a final concentration of 3.5×10¹⁰, 3.5×10⁹, 3.5×10⁸, and 3.5×10⁷ nanoparticles/mL (based on NTA) or spiked with an equivalent volume of control sample (MQ-water treated the same way as the sucrose solutions and showing nanoparticle numbers below the quantification limit of NTA). All samples of the 3.5×10¹⁰ nanoparticles/mL NPI level were then transferred to sterile 2R vials (fill volume 1 mL) and each aliquot (N=1) was measured at least one day after preparation (to), after 2, 8 and 14 weeks of storage (t_{2w}, t_{8w}, t_{14w}) at 2-8 °C, 25 °C or 40 °C.

Furthermore, samples of all four NPI levels were stored at 40 °C for one week for a concentration-dependency study. Sample handling was performed under laminar airflow conditions.

In order to evaluate the effects of the NPI on the stability of the monoclonal antibodies, samples were evaluated visually, by micro-flow imaging (MFI), nanoparticle tracking analysis and size-exclusion chromatography (SEC).

For visual inspection according to the European Pharmacopoeia, the vials were independently tested by two trained examiners for presence or absence of visible particles or turbidity under gentle, manual, radial agitation for 5 s in front of a white background and for 5 s in front of a black background.

For Micro-flow imaging (MFI), an MFI5200 system (ProteinSimple, Santa Clara, CA, USA) equipped with a 100-µm flow cell and controlled by the MFI View System Software (MVSS) version 2-R2.6.1.20.1915 was used. The system was flushed with 10 mL purified water at maximum flow rate and flow cell cleanliness was checked visually between measurements. The respective formulation buffer was used as a blank to perform illumination optimization prior to each measurement. Samples of 0.5 mL with a pre-run volume of 0.2 mL were analysed at a flow rate of 0.17 mL/min and a fixed camera rate (not adjustable by the user), leading to a sampling efficiency of about 80-85 %.

Size-exclusion chromatography (SEC) was performed on a TSK Gel 4000 SWXL column (300 mm × 7.8 mm) (Tosoh Bioscience, Montgomeryville, USA) and an Agilent 1200 high-performance liquid chromatography system (Agilent Technologies, Palo Alto, USA) coupled to an ultraviolet (UV) detector set at 280 nm. The mobile phase was composed of 100 mM sodium phosphate, 100 mM sodium sulfate at a pH of 7.0. The flow rate was set to 0.6 mL/min. Samples were centrifuged at 10,000 g for 3 min, kept at 2-8 °C and injected in duplicates of each 25 µL. The sample recovery was calculated as the total peak area relative to the control-spiked sample at to. Peaks with a retention time above 20 min were buffer related and not considered. The monomer peak retention time was at 17.5 min. Peaks with a shorter retention time than the monomer were regarded as high molecular weight (HMW) species. In contrast, peaks with a longer retention time than the monomer were regarded as low molecular weight (LMW) species. Contents of monomer, HMW- and LMW species are given as percentages relative to the total sample recovery.

### Results

The NPI negatively affect the stability of all four tested monoclonal antibodies (mAbs), although to different extents; i.e. all four showed degradation upon exposure to NPI particles, which was more pronounced as compared to the control sample. Table 1 summarizes and ranks the observed effects of the NPIs on the stability of the tested antibodies. A higher score (sum of +'s) correlates to a more pronounced mAb degradation compared to the other tested mAbs.

Rituximab was affected to the lowest extent by the presence of the NPIs, showing immediate formation of high numbers of nm-sized particles as the main degradation product. Cetuximab similarly showed immediate formation of high numbers of nm-sized particles as the main degradation product, but was overall degraded more severely than rituximab. Trastuzumab showed formation of high numbers of µm-sized particles as the main degradation product and the appearance of turbidity. Among the tested mAbs, infliximab was degraded to the greatest extent, showing high numbers of nm- and µm-sized particles as the main degradation product as well as the appearance of turbidity.

Furthermore, the stability of trastuzumab and infliximab was affected directly after spiking NPI. In contrast, degradation of rituximab and cetuximab was observed only after 14 weeks at elevated temperatures. Moreover, the stability of rituximab and infliximab was affected by NPI concentrations that are potentially present in final drug products. The stability of trastuzumab, however, was only affected at elevated NPI concentrations.

**Table 1: Influence of NPIs on stability parameters of mAbs (relative to control)**

| **Parameter** | **Method** | **trastuzumab** | **rituximab** | **infliximab** | **cetuximab** |
|---|---|---|---|---|---|
| Visible particles | Visual inspection | + | - | + | - |
| Turbidity | Visual inspection | ++ | - | ++ | + |
| µm-particles | MFI | ++ | + | ++ | + |
| nm-particles | NTA | - | ++ | ++ | ++ |
| HMW species | SEC | - | - | - | + |
| Sample recovery | SEC | + | - | + | - |
| LMW species | SEC | - | + | + | - |
| | **Sum of +'s** | 6 | 4 | 9 | 5 |

| | | | | | |
|---|---|---|---|---|---|
| - = not affected + = affected at high concentrations of NPIs and/or delayed effect ++ = highly and immediately affected and/or affected at NPIs concentrations potentially present in drug products | | | | | |

In contrast, formulation buffer controls did not show instabilities in any of the tested parameters and the pH values measured immediately after sample preparation were within 0.1 pH units from those of the corresponding control samples. It can thus be excluded that the observations are purely due to differences in formulation buffer composition or a result of excipient instabilities and/or pH shifts.

It is concluded that the presence of NPI in (bio-)pharmaceutical or diagnostic compositions poses a threat to the stability of mAbs. Additionally, NPI may have unwanted immunological consequences, since the NPI particles were shown to contain a high content of (1-3)-β-glucan, which is an immune-modulating molecule.

## Claims

1. A method of preparing ultrapure sucrose, the method comprising the steps of:
(b) providing a quantity of a first sucrose comprising water-insoluble nanoparticulate impurities (NPI) at a level of 10⁶ nanoparticles per gram of sucrose or higher, wherein the nanoparticles have a particle size ranging from 20 nm to 500 nm, comprise a (1-3)-β-glucan, and exhibit endotoxin-like properties;
wherein prior to step (b), a step (a) is conducted of obtaining or determining the level of NPI in the first sucrose,
(c) reducing the level of NPI to 10⁵ nanoparticles per gram of sucrose or lower by
(i) separating at least some of the NPI from the sucrose, optionally by filtration, centrifugation, crystallisation or chromatography of a solution of said sucrose; or
(ii) diluting the first sucrose with a second sucrose, the second sucrose comprising NPI at a level below 10⁵ nanoparticles per gram of sucrose, and optionally
(d) concentrating the sucrose solution of step (i) to a concentration of 10 % (w/v) or higher, or drying the sucrose solution such as to obtain dry solid sucrose.

2. The method of claim 1, wherein the NPI particles have a particle size distribution in an aqueous solution **characterised by** a D50 value from 80 nm to 300 nm as measured by nanoparticle tracking analysis (NTA), in particular from 100 nm to 200 nm.

3. The method of claims 1 or 2, wherein the NPI comprise:
(a) a dextran, wherein the dextran is optionally crosslinked and/or exhibits a mean molecular mass from 10 kDa to 100 kDa,
(b) an organic colourant, and/or
(c) an inorganic compound selected from silicium, aluminium, calcium, magnesium, phosphorus, sulphur, potassium, iron, or any combination of any of these.

4. The method of any one of the preceding claims, wherein step (a) is conducted by nanoparticle tracking analysis (NTA).

5. The method of any one of the preceding claims, wherein step (c) is conducted such as to reduce the level of NPI to not more than 10³ nanoparticles per gram of sucrose, or not more than 10² nanoparticles per gram of sucrose; or such that the sucrose is substantially free of NPI, as determined by light-scattering based analytical techniques, such as dynamic light scattering (DLS) or nanoparticle tracking analysis (NTA).

6. The method of any one of the preceding claims, wherein the filtration is performed as ultrafiltration or ultradiafiltration of an aqueous solution of the first sucrose, optionally through a filter with a pore size of 0.02 µm or smaller or through a membrane with a cut-off of 30 kDa or smaller; and/or
wherein the centrifugation is performed as ultracentrifugation of an aqueous solution of the first sucrose.

7. Sucrose obtainable by the method of any one of the preceding claims.

8. Solid, crystalline sucrose comprising water-insoluble nanoparticulate impurities (NPI) at a level of not more than 10⁵ nanoparticles per gram of sucrose as determined by nanoparticle tracking analysis (NTA), preferably not more than 10³ nanoparticles per gram of sucrose, and more preferably not more than 10² nanoparticles per gram of sucrose.

9. Use of the sucrose of claims 7 or 8 for the preparation of a pharmaceutical or diagnostic composition comprising an active ingredient, wherein the active ingredient is preferably
(a) a peptide, a protein, a conjugate comprising a peptide or protein, a lipopeptide, or a lipoprotein;
(b) prone to aggregation and/or degradation; and/or
(c) sensitive to heat, mechanical stress, oxygen, acidic and/or basic conditions.

10. A method of preparing a pharmaceutical or diagnostic composition comprising an active ingredient and sucrose, the method comprising the steps of
(a) obtaining or determining the level of NPI in the sucrose, optionally by nanoparticle tracking analysis (NTA);
(b) if the level of NPI in the sucrose is 10⁶ nanoparticles per gram of sucrose or higher, or if the level of NPI in the sucrose is such that it would result in a level of NPI of 10⁵ nanoparticles per gram of composition or higher, reducing the level of NPI to 10⁵ nanoparticles per gram of sucrose or lower by
(i) separating at least some of the NPI from the sucrose, optionally by filtration, centrifugation, crystallisation or chromatography; or
(ii) diluting the first sucrose with a second sucrose, the second sucrose comprising NPI at a level below 10⁵ nanoparticles per gram of sucrose; and
(c) preparing the pharmaceutical or diagnostic composition incorporating the sucrose.

11. The method of claim 10, wherein a plurality of consecutive batches of said pharmaceutical or diagnostic composition are prepared, and wherein each of these batches is prepared in the manner of claim 10, optionally using different batches of sucrose.

12. The method of claims 10 to 11, wherein the pharmaceutical or diagnostic composition is in the form of
(a) a powder or a lyophilised solid foam for reconstitution, or
(b) an aqueous liquid.

13. A pharmaceutical or diagnostic composition as obtained by the method of claims 10 to 12 for use in the diagnosis or therapy of a human or a non-human animal.

14. The pharmaceutical or diagnostic composition for use according to claim 13, wherein the active ingredient is
(a) a peptide, a protein, a conjugate comprising a peptide or protein, a lipopeptide, or a lipoprotein;
(b) prone to aggregation and/or degradation; and/or
(c) sensitive to heat, mechanical stress, oxygen, acidic and/or basic conditions.

15. The pharmaceutical or diagnostic composition for use according to claim 14, wherein the
active ingredient is a monoclonal antibody, and/or
wherein the sucrose is substantially free of NPI as determined by nanoparticle tracking analysis (NTA).

## Patentansprüche

1. Verfahren zur Herstellung von ultrareiner Saccharose, wobei das Verfahren die Schritte umfasst:
(b) Bereitstellen einer Menge einer ersten Saccharose, die wasserunlösliche nanopartikuläre Verunreinigungen (NPI) in einem Niveau von 10⁶ Nanopartikel pro Gramm Saccharose oder höher enthält, wobei die Nanopartikel eine Partikelgröße im Bereich von 20 nm bis 500 nm haben, ein (1-3)-β-Glucan enthalten und endotoxinartige Eigenschaften aufweisen;
wobei vor Schritt (b) ein Schritt (a) zum Erhalten oder Bestimmen des Niveaus an NPI in der ersten Saccharose durchgeführt wird,
(c) Reduzieren des Niveaus an NPI auf 10⁵ Nanopartikel pro Gramm Saccharose oder darunter durch
(i) Abtrennen von mindestens einigen der NPI von der Saccharose, optional durch Filtration, Zentrifugation, Kristallisation oder Chromatographie einer Lösung der Saccharose; oder
(ii) Verdünnen der ersten Saccharose mit einer zweiten Saccharose, wobei die zweite Saccharose NPI in einem Niveau unter 10⁵ Nanopartikel pro Gramm Saccharose enthält, und optional
(d) Konzentrieren der Saccharoselösung aus Schritt (i) auf eine Konzentration von 10 % (Gewicht/Volumen) oder höher, oder Trocknen der Saccharoselösung, um trockene feste Saccharose zu erhalten.

2. Verfahren nach Anspruch 1, wobei die NPI-Partikel in einer wässrigen Lösung eine Partikelgrößenverteilung haben, welche **gekennzeichnet ist durch** einen D50-Wert von 80 nm bis 300 nm, gemessen mittels Nanopartikel-Tracking-Analyse (NTA), insbesondere von 100 nm bis 200 nm.

3. Verfahren nach Anspruch 1 oder 2, wobei die NPI enthalten:
(a) ein Dextran, wobei das Dextran optional vernetzt ist und/oder eine mittlere Molekülmasse von 10 kDa bis 100 kDa aufweist,
(b) ein organisches Färbemittel und/oder
(c) eine anorganische Verbindung ausgewählt aus Silicium, Aluminium, Calcium, Magnesium, Phosphor, Schwefel, Kalium, Eisen oder jedweder Kombination derselbigen.

4. Verfahren nach einem der vorhergehenden Ansprüche, wobei Schritt (a) durch Nanopartikel-Tracking-Analyse (NTA) durchgeführt wird.

5. Verfahren nach einem der vorhergehenden Ansprüche, wobei Schritt (c) derart durchgeführt wird, dass das Niveau an NPI reduziert wird auf nicht mehr als 10³ Nanopartikel pro Gramm Saccharose, oder nicht mehr als 10² Nanopartikel pro Gramm Saccharose; oder derart, dass die Saccharose im Wesentlichen frei von NPI ist, wie durch Analysetechniken auf Lichtstreuungsbasis bestimmt wird, wie dynamische Lichtstreuung (DLS) oder Nanopartikel-Tracking-Analyse (NTA).

6. Verfahren nach einem der vorhergehenden Ansprüche, wobei die Filtration als Ultrafiltration oder Ultradiafiltration einer wässrigen Lösung der ersten Saccharose durchgeführt wird, optional durch einen Filter mit einer Porengröße von 0,02 µm oder kleiner, oder durch eine Membran mit einer Ausschlussgröße von 30 kDa oder kleiner; und/oder
wobei das Zentrifugieren als Ultrazentrifugieren einer wässrigen Lösung der ersten Saccharose durchgeführt wird.

7. Saccharose, die nach dem Verfahren gemäß einem der vorhergehenden Ansprüche erhältlich ist.

8. Feste kristalline Saccharose, enthaltend wasserunlösliche nanopartikuläre Verunreinigungen (NPI) auf einem Niveau von nicht mehr als 10⁵ Nanopartikel pro Gramm Saccharose, gemessen mittels Nanopartikel-Tracking-Analyse (NTA), vorzugsweise nicht mehr als 10³ Nanopartikel pro Gramm Saccharose, und bevorzugter nicht mehr als 10² Nanopartikel pro Gramm Saccharose.

9. Verwendung der Saccharose nach den Ansprüchen 7 oder 8 zur Herstellung einer pharmazeutischen oder diagnostischen Zusammensetzung, die einen aktiven Bestandteil umfasst, wobei der aktive Bestandteil vorzugsweise
(a) ein Peptid, ein Protein, ein Konjugat, enthaltend ein Peptid oder Protein, ein Lipopeptid oder ein Lipoprotein;
(b) anfällig für Aggregation und/oder Abbau ist; und/oder
(c) empfindlich gegenüber Wärme, mechanischer Beanspruchung, Sauerstoff, sauren und/oder basischen Bedingungen ist.

10. Verfahren zur Herstellung einer pharmazeutischen oder diagnostischen Zusammensetzung, die einen aktiven Bestandteil und Saccharose enthält, wobei das Verfahren die Schritte umfasst
(a) Erhalten oder Bestimmen des Niveaus an NPI in der Saccharose, optional durch Nanopartikel-Tracking-Analyse (NTA);
(b) falls das Niveau an NPI in der Saccharose 10⁶ Nanopartikel pro Gramm Saccharose oder höher ist, oder falls das Niveau an NPI in der Saccharose so ist, dass es zu einem Niveau an NPI von 10⁵ Nanopartikel pro Gramm der Zusammensetzung oder höher führen würde, Reduzieren des Niveaus an NPI auf 10⁵ Nanopartikel pro Gramm Saccharose oder niedriger durch
(i) Abtrennen von mindestens einigen der NPI von der Saccharose, optional durch Filtration, Zentrifugation, Kristallisation oder Chromatographie; oder
(ii) Verdünnen der ersten Saccharose mit einer zweiten Saccharose, wobei die zweite Saccharose NPI in einem Niveau unter 10⁵ Nanopartikel pro Gramm Saccharose enthält; und
(c) Herstellen der pharmazeutischen oder diagnostischen Zusammensetzung durch Einarbeitung der Saccharose.

11. Verfahren nach Anspruch 10, wobei eine Vielzahl aufeinanderfolgender Chargen der pharmazeutischen und diagnostischen Zusammensetzung hergestellt wird, und wobei jede dieser Chargen in der Weise von Anspruch 10 hergestellt wird, optional unter Verwendung unterschiedlicher Saccharose-Chargen.

12. Verfahren nach den Ansprüchen 10 bis 11, wobei die pharmazeutische oder diagnostische Zusammensetzung vorliegt in Form
(a) eines Pulver oder lyophilisierten festen Schaumes zur Rekonstitution, oder
(b) einer wässrigen Flüssigkeit.

13. Pharmazeutische oder diagnostische Zusammensetzung, wie sie durch das Verfahren nach den Ansprüchen 10 bis 12 erhalten wird, zur Verwendung in der Diagnostik oder Therapie eines Menschen oder nicht-menschlichen Tieres.

14. Pharmazeutische oder diagnostische Zusammensetzung zur Verwendung nach Anspruch 13, wobei der aktive Bestandteil
(a) ein Peptid, ein Protein, ein Konjugat, enthaltend ein Peptid oder Protein, ein Lipopeptid oder ein Lipoprotein;
(b) anfällig für Aggregation und/oder Degradierung ist; und/oder
(c) empfindlich gegenüber Wärme, mechanischer Beanspruchung, Sauerstoff, sauren und/oder basischen Bedingungen ist.

15. Pharmazeutische oder diagnostische Zusammensetzung zur Verwendung nach Anspruch 14, wobei der aktive Bestandteil ein monoklonaler Antikörper ist, und/oder wobei die Saccharose im Wesentlichen frei von NPI ist, gemessen mittels Nanopartikel-Tracking-Analyse (NTA).

## Revendications

1. Procédé de préparation de saccharose ultra-pur, le procédé comprenant les étapes suivantes :
(b) fournir une quantité d'un premier saccharose comprenant des impuretés nano-particulaires (NPI) insolubles dans l'eau à un niveau de 10⁶ nanoparticules par gramme de saccharose ou plus, les nanoparticules ayant une taille de particule dans la gamme de 20 nm à 500 nm, comprenant un (l-3)-β-glucane et présentent des propriétés de type endotoxine ;
avant l'étape (b), une étape (a) étant réalisée pour obtenir ou déterminer le niveau de NPI dans le premier saccharose,
(c) réduire le niveau de NPI à 10⁵ nanoparticules par gramme de saccharose ou moins par
(i) séparation d'au moins une partie des NPI du saccharose, éventuellement par filtration, centrifugation, cristallisation ou chromatographie d'une solution dudit saccharose ; ou
(ii) dilution du premier saccharose avec un deuxième saccharose, le deuxième saccharose comprenant des NPI à un niveau inférieur à 10 nanoparticules par gramme de saccharose, et éventuellement
(d) concentrer la solution de saccharose de l'étape (i) à une concentration de 10 % (p/v) ou plus, ou sécher la solution de saccharose de manière à obtenir du saccharose solide sec.

2. Procédé selon la revendication 1, les particules de NPI ayant une distribution granulométrique dans une solution aqueuse **caractérisée par** une valeur D50 de 80 nm à 300 nm telle que mesurée par analyse de suivi de nanoparticules (NTA), notamment de 100 nm à 200 nm.

3. Procédé selon les revendications 1 ou 2, les NPI comprenant :
(a) un dextrane, le dextrane étant éventuellement réticulé et/ou présentant une masse moléculaire moyenne de 10 kDa à 100 kDa,
(b) un colorant organique, et/ou
(c) un composé minéral choisi parmi le silicium, l'aluminium, le calcium, le magnésium, le phosphore, le soufre, le potassium, le fer ou toute combinaison de ceux-ci.

4. Procédé selon l'une quelconque des revendications précédentes, l'étape (a) étant réalisée par analyse de suivi de nanoparticules (NTA).

5. Procédé selon l'une quelconque des revendications précédentes, l'étape (c) étant réalisée de manière à réduire le niveau de NPI à pas plus de 10³ nanoparticules par gramme de saccharose, ou pas plus de 10² nanoparticules par gramme de saccharose ; ou de sorte que le saccharose soit sensiblement exempt de NPI, tel que déterminé par des techniques analytiques basées sur la diffusion de la lumière, telles que la diffusion dynamique de la lumière (DLS) ou l'analyse de suivi de nanoparticules (NTA).

6. Procédé selon l'une des revendications précédentes, la filtration étant effectuée sous la forme d'une ultrafiltration ou d'une ultradiafiltration d'une solution aqueuse du premier saccharose, éventuellement à travers un filtre ayant une taille de pores de 0,02 µm ou moins ou à travers une membrane ayant une coupure de 30 kDa ou moins ; et/ou
la centrifugation étant effectuée sous la forme d'une ultracentrifugation d'une solution aqueuse du premier saccharose.

7. Saccharose pouvant être obtenu par le procédé selon l'une quelconque des revendications précédentes.

8. Saccharose cristallin solide comprenant des impuretés nano-particulaires (NPI) insolubles dans l'eau à un niveau ne dépassant pas 10⁵ nanoparticules par gramme de saccharose tel que déterminé par analyse de suivi de nanoparticules (NTA), de préférence pas plus de 10³ nanoparticules par gramme de saccharose, et plus préférablement pas plus de 10² nanoparticules par gramme de saccharose.

9. Utilisation du saccharose des revendications 7 ou 8 pour la préparation d'une composition pharmaceutique ou diagnostique comprenant un principe actif, le principe actif étant de préférence
(a) un peptide, une protéine, un conjugué comprenant un peptide ou une protéine, un lipopeptide ou une lipoprotéine ;
(b) sujet à l'agrégation et/ou à la dégradation ; et/ou
(c) sensible à la chaleur, aux contraintes mécaniques, à l'oxygène, aux conditions acides et/ou basiques.

10. Procédé de préparation d'une composition pharmaceutique ou diagnostique comprenant un principe actif et du saccharose, le procédé comprenant les étapes suivantes :
(a) obtenir ou déterminer le niveau de NPI dans le saccharose, éventuellement par analyse de suivi de nanoparticules (NTA) ;
(b) si le niveau de NPI dans le saccharose est de 10⁶ nanoparticules par gramme de saccharose ou plus, ou si le niveau de NPI dans le saccharose est tel qu'il en résulterait un niveau de NPI de 10⁵ nanoparticules par gramme de composition ou plus, réduire le niveau de NPI à 10 nanoparticules par gramme de saccharose ou moins par
(i) séparation d'au moins une partie des NPI du saccharose, éventuellement par filtration, centrifugation, cristallisation ou chromatographie ; ou
(ii) dilution du premier saccharose avec un deuxième saccharose, le deuxième saccharose comprenant des NPI à un niveau inférieur à 10⁵ nanoparticules par gramme de saccharose ; et
(c) préparer la composition pharmaceutique ou diagnostique incorporant le saccharose.

11. Procédé selon la revendication 10, une pluralité de lots consécutifs de ladite composition pharmaceutique ou diagnostique étant préparés, et chacun de ces lots étant préparé à la manière de la revendication 10, éventuellement à l'aide de différents lots de saccharose.

12. Procédé selon les revendications 10 à 11, la composition pharmaceutique ou diagnostique se présentant sous la forme
a) d'une poudre ou d'une mousse solide lyophilisée à reconstituer, ou
(b) d'un liquide aqueux.

13. Composition pharmaceutique ou diagnostique telle qu'obtenue par le procédé selon les revendications 10 à 12 et destinée à être utilisée dans le diagnostic ou la thérapie d'un animal humain ou non humain.

14. Composition pharmaceutique ou diagnostique destinée à être utilisée selon la revendication 13, le principe actif étant
(a) un peptide, une protéine, un conjugué comprenant un peptide ou une protéine, un lipopeptide ou une lipoprotéine ;
(b) sujet à l'agrégation et/ou à la dégradation ; et/ou
(c) sensible à la chaleur, aux contraintes mécaniques, à l'oxygène, aux conditions acides et/ou basiques.

15. Composition pharmaceutique ou diagnostique destinée à être utilisée selon la revendication 14, le principe actif étant un anticorps monoclonal, et/ou
le saccharose étant sensiblement exempt de NPI comme déterminé par analyse de suivi de nanoparticules (NTA).
